# EUROPÄISCHE PATENTSCHRIFT

(11) **EP 1 857 375 B1**
(45) Veröffentlichungstag und Bekanntmachung des Hinweises auf die Patenterteilung: **07.01.2009**
(21) Anmeldenummer: 07009609.4
(22) Anmeldetag: 14.05.2007
(51) Int. Cl.: B65D 55/02, B65D 55/06

(54) **Behälterverschluss mit Verschlussplombe für Kunststoffbehälter**
Container lid with seal for plastic containers
Fermeture de récipient doté d'un plomb de fermeture pour récipient en plastique

(30) Priorität: 18.05.2006 DE 202006007933 U
(43) Veröffentlichungstag der Anmeldung: 21.11.2007
(73) Patentinhaber: Ringoplast GmbH, 49824 Ringe-Neugnadenfeld (DE)
(72) Erfinder: Johannink, Hendrik, 48527 Nordhorn (DE); Kapell, Frank, 48455 Bad Bentheim (DE)
(74) Vertreter: Meinke, Dabringhaus und Partner GbR

(56) Entgegenhaltungen:
- WO-A-20/04068993
- BE-A- 899 151
- GB-A- 132 565
- US-A- 1 802 214

## Beschreibung

Die Erfindung richtet sich auf einen Behälterverschluss, insbesondere zur Anbringung einer den Schließzustand sicherstellenden, nicht zerstörungsfrei entfernbaren Verschlussplombe, insbesondere bei Kunststoffbehältern.

Aus einer nicht vorveröffentlichten Gebrauchsmusteranmeldung der Anmelderin ist ein Isolierbehälter mit Verschlussplombe bekannt, wobei die Plombe an einer Seite einen Plombenkopf aufweist und an der im Material des Behälters eingreifenden anderen Seite Wulst- oder Widerhakenelemente. Zu diesem Stand der Technik gehört auch die DE-44 37 694-A1, bei der der Deckel im Randbereich Durchgangsbohrungen ebenso aufweist wie der Auflagerand des Behälters, wobei durch diese Bohrungen Verankerungsstifte mit Sollbruchstellen hindurchgesteckt werden. Eine ähnliche Lösung zeigt auch die DE-27 35 151-A1. Sicherungsplomben mit Spreizschenkeln und Sollbruchstellen offenbart auch das DE-299 07 481-U, um nur einige Lösungen aus dem Stand der Technik zu nennen.

Weitere Verschlusslösungen für Warenversandbehälter sind beispielsweise in der DE 196 05 377 C2, der DE 28 32 069 C2, der DE 198 22 029 B4, der US 5,791,702, der OS 64 43 508 B1 und der WO 2004/068993 beschrieben.

Werden die Plomben nach dem Stand der Technik entfernt, ist häufig nicht mehr sichergestellt, dass die so ausgestatteten Behälter auch wiederum sicher verschließbar sind, wenn auch ohne Plombe. Umgekehrt weisen häufig Clips- oder Sperrverschlüsse an Behältern nicht die Möglichkeit der Anbringung einer Plombe auf.

Hier setzt die Erfindung an, deren Ziel darin besteht, einen Behälterverschluss zu schaffen, mit dem zum einen ein sicheres Verbinden zwischen Behälterdeckel einerseits und Behälterkörper andererseits gewährleistet ist, wobei dieser Verschluss auch die Möglichkeit bieten soll, Sicherungsplomben anzubringen.

Mit einem Behälterverschluss der eingangs bezeichneten Art wird diese Aufgabe gemäß der Erfindung gelöst durch
- eine Sperrlasche an der Unterseite des Behälterdeckels im Randbereich,
- eine im oberen Randbereich des Behälters der Sperrlasche zugeordnete Durchtrittsausnehmung,
- eine schwenkbare Schließlasche im Randbereich des Behälters, wobei die Schließlasche einen Eingriffssteg zum Eingriff in eine korrespondierende Ausnehmung der Sperrlasche und eine Klemmraste zur Fixierung der Sperrlasche in Schließstellung aufweist sowie durch
- eine Plombendurchtrittsöffnung in der Schließlasche.

Durch diese Sperrlaschen-/Schließlaschenkonstruktion ist zum einen ein vergleichsweise einfacher sicherer Verschluss zwischen Behälterdeckel einerseits und Behälterkörper andererseits gewährleistet, zum anderen lässt sich mit Hilfe der Schließlasche und der entsprechenden Durchtrittsbohrung auch eine Sicherungsplombe mühelos anbringen.

Nicht zuletzt um bereits vorhandene Behälter in ihrem Nutzwert aufzuwerten sieht die Erfindung vor, dass die Sperrlasche und/oder die Schließlasche als an den Behälterelementen dauerhaft fixierbare, selbstständige Bauteile ausgebildet sind.

So kann beispielsweise an einer dem späteren Verschweißen mit dem Behälterdeckel dienenden Tragplatte die Sperrlasche einstückig angespritzt sein, so dass Behälterdeckel entsprechend nachrüstbar sind.

Um auch die Schließlasche nachrüsten zu können, kann erfindungsgemäß vorgesehen sein, dass die Schließlasche an einem ggf. nachträglich am Behälter fixierbaren Grundkörper schwenkbar angelenkt ist.

Weitere zweckmäßige Ausgestaltungen ergeben sich aus den Unteransprüchen. Dabei kann z.B. vorgesehen sein, dass die Schließlasche und der behälterfeste Grundkörper eine Durchgangsbohrung zum Durchtritt der Verschlussplombe aufweist. Damit lässt sich nach dem Schließen eine Verschlussplombe zwischen Schließlasche und Grundkörper positionieren, so dass nach Zerstörung der Verschlussplombe sofort erkennbar ist, dass der Verschluss geöffnet wurde oder zumindest am Verschluss Manipulationen vorgenommen wurden.

Vorteilhaft ist nach der Erfindung der Behälterrand querschnittlich als nach außen offenes U geformt, wobei der Grundkörper am U-Steg fixiert ist. Damit ist es möglich, den Gesamtverschluss in der Kontur des Behälters unterzubringen, ohne dass der Verschluss den Behälter nach außen in irgendeiner Form überragt.

Nach der Erfindung kann auch vorgesehen sein, dass der Grundkörper eine gestufte Lasche mit einer Durchtrittsbohrung für die Plombe aufweist, die außenrandseitig einen Fixiersteg für eine Klemmwulst an der schwenkbaren Schließlasche bildet.

Durch diese Stufung wird gleichzeitig zwischen Stufenunterseite einerseits und U-Steg-Oberfläche am Behälter andererseits ein Eintrittsraum geschaffen für die Widerhaken der Verschlussplombe, d.h. der Behälter selbst muss mit keiner Durchtrittsbohrung für die Plombe ausgerüstet werden, so dass das Behälterinnere hiervon völlig unbehelligt bleibt.

Wird diese Gestaltung vorgenommen, sieht die Erfindung schließlich noch in weiterer Ausgestaltung vor, dass der die Klemmwulst tragende Fixiersteg als Abschlusswandung für an der Plombe vorgesehene Widerhaken ausgestaltet ist. Mit dieser Gestaltung wird der Raum für die Widerhaken in der Verschlusslage völlig von der Abschlusswandung umschlossen, so dass die Widerhaken nicht etwa mittels eines Werkzeuges vorübergehend zusammengedrückt werden können, derart, dass die Plombe zerstörungsfrei entfernt werden könnte. Dies wird mit der Abschlusswandung ausdrücklich verhindert.

Weitere Merkmale, Einzelheiten und Vorteile der Erfindung ergeben sich aufgrund der nachfolgenden Beschreibung sowie anhand der Zeichnung. Diese zeigt in
- Fig. 1: eine räumliche Darstellung eines Ausrisses aus einem Behälter mit einem erfindungsgemäßen Behälterverschluss in teilweiser Öffnungsstellung,
- Fig. 2: eine Aufsicht auf den Behälterrand mit geschlossenem Verschluss, teilweise aufgebrochen,
- Fig. 3: einen Schnitt etwa nach Linie III-III in Fig. 2 sowie in
- Fig. 4: drei Stellungen der Schließlasche relativ zu den am Behälter zu befestigenden Grundkörper.

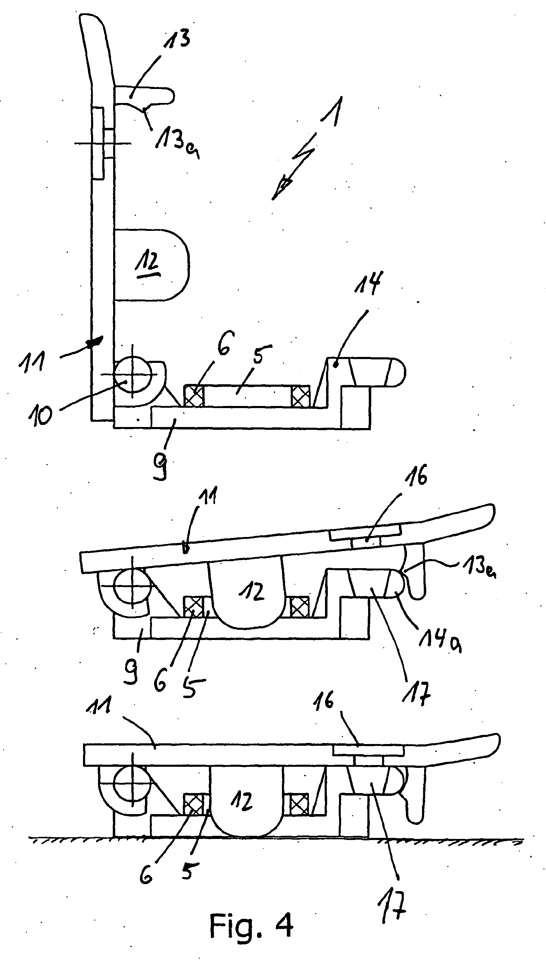

Der allgemein mit 1 bezeichnete, in Fig. 4 in verschiedenen Positionen dargestellte Behälterverschluss dient zur Fixierung eines Behälterdeckels 2 an einem Aufnahmebehälter, allgemein mit 3 bezeichnet, z.B. zum Transport von vorbestellten Artikeln, zum Transport von gekühlen Lebensmitteln od. dgl., wobei der Einsatz nicht auf ein besonderes Gebiet beschränkt ist.

Wesentlich für den Behälterverschluss 1 ist, dass er so gestaltet ist, dass zum einen die Fixierung des Behälterdeckels 2 am Behälter 3 gewährleistet ist, gleichzeitig soll aber auch eine Möglichkeit bestehen, den Verschluss gegen unautorisiertes Öffnen durch eine Plombe zu schützen, derart, dass bei einem nicht autorisierten Öffnen dies vom Benutzer sofort erkannt werden kann.

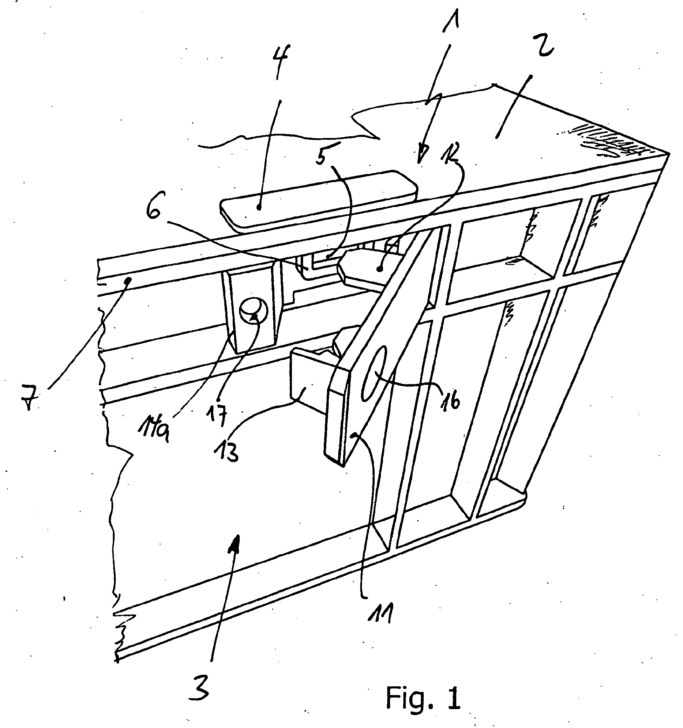

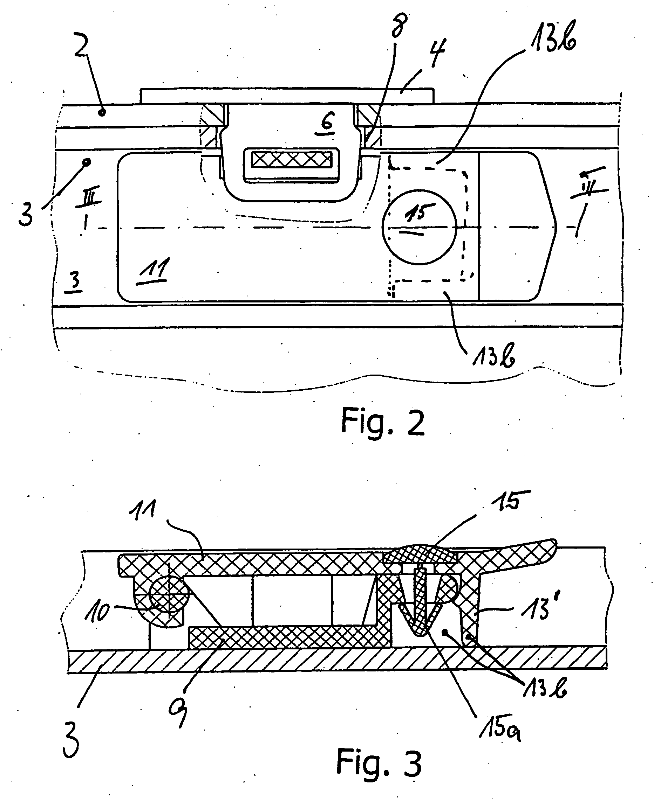

Um sowohl das Schließen als auch das Anbringen einer Plombe zu gewährleisten, ist am Behälterdeckel 1 im dargestellten Beispiel über eine Tragplatte 4 eine mit einer Durchtrittsöffnung 5 versehene Sperrlasche 6 vorgesehen, die durch eine in Fig. 1 nicht näher dargestellte Öffnung im oberen Rand 7 des Behälters 3 beim Schließen des Deckels 2 hindurchtritt, wie dies in den Fig. 1 und 2 angedeutet ist. Die Durchtrittsöffnung für die Sperrlasche 6 ist in Fig. 2 mit 8 bezeichnet.

An einem mit 9 bezeichneten Grundkörper ist über ein Drehgelenk 10 eine Schließlasche 11 am Behälter 3 befestigt, wobei die Schließlasche 11 einen Eingriffssteg 12 aufweist, der in der Schließlage in die Ausnehmungen 5 an der Sperrlasche 6 eingreift bzw. diese durchsetzt und so verriegelt, wie sich dies beispielsweise aus Fig. 4 ergibt.

Etwa im vorderen, dem Scharnier 10 abgewandten Bereich der Schließlasche 11 weist diese eine Klemmraste 13 auf, die zur Fixierung der Schließlasche 11 in der Verriegelungsposition dient. Zur Verriegelung weist die Klemmraste 13 einen nach innen weisenden Klemmwulst 13a auf, während der Grundkörper 9 einen gestuften Außenrandvorsprung als Fixiersteg 14 aufweist, der außenrandseitig an seinem vorderen freien Ende 14a vom Klemmnocken 13a untergriffen wird, wie dies die Verriegelungsposition zeigende Darstellung der Fig. 4 zeigt.

Zur Anbringung einer in Fig. 3 mit 15 bezeichneten Sicherungsplombe ist in der Schließlasche 11 eine Durchtrittsbohrung 16 vorgesehen, ebenso wie im gestuften Fixiersteg 14 des Grundkörpers 9. Diese Durchsetzbohrung ist mit 17 bezeichnet.

In den Fig. 2 und 3 ist noch zum Teil gestrichelt dargestellt, dass die Klemmraste 13' als Abschlussrandung gestaltet sein kann, die den Widerhakenteil 15a der Plombe 15 in der Verschlusslage (Fig. 3) so umschließt, dass die Widerhaken nicht mit einem Werkzeug od. dgl. zerstörungsfrei zusammengedrückt und damit die Plombe wiederum zerstörungsfrei entnommen werden kann, der Inhalt des Behälters manipuliert und anschließend der Verschluss geschlossen und die Plombe wieder eingeführt werden kann. Dieser in Fig. 2 gestrichelt angedeutete, die Plombe 15 umschließende Randbereich ist mit 13b bezeichnet.

Natürlich ist das beschriebene Ausführungsbeispiel der Erfindung noch in vielfacher Hinsicht abzuändern, ohne den Grundgedanken zu verlassen. So kann beispielsweise die Sperrlasche 6 einstückig bei der Herstellung des Deckels 2 an diesem angespritzt sein. Auch kann der Schwenksockel mit dem Drehgelenk 10 in den Nutengrund des Behälterrandes einstückig eingeformt sein, so dass später nur die Schließlasche 11 einzuclipsen ist.

## Patentansprüche

1. Behälterverschluss, insbesondere zur Anbringung einer im Schließzustand sicher stellenden, nicht zerstörungsfrei entfernbaren Verschlussplombe, insbesondere bei Kunststoffbehältern, mit einer Sperrlasche (6), einer im oberen Randbereich des Behälters der Sperrlasche (6) zugeordneten Durchtrittsausnehmung (8), einer schwenkbaren Schließlasche (11), wobei die Schließlasche (11) einen Eingriffssteg (12) aufweiset, sowie einer Plombendurchtrittsöffnung (16),
**dadurch gekennzeichnet,**
**dass** die Sperrlasche (6) an der Unterseite des Behälterdeckels (2) im Randbereich angeordnet ist, und dass die Schließlasche (11) im Randbereich des Behälters vorgesehen ist und einen Eingriffssteg (12) zum Eingriff in eine korrespondierende Ausnehmung (5) der Sperrlasche (6) und eine Klemmraste (13) zur Fixierung der Sperrlasche (6) in Schließstellung aufweist, wobei die Plombendurchtrittsöffnung (16) in der Schließlasche (11) vorgesehen ist.

2. Behälterverschluss nach Anspruch 1,
**dadurch gekennzeichnet,**
**dass** die Sperrlasche (6) und/oder die Schließlasche (11) als an den Behälterelementen (2,3) dauerhaft fixierbare, selbstständige Bauteile ausgebildet sind.

3. Behälterverschluss nach Anspruch 2,
**dadurch gekennzeichnet,**
**dass** die Schließlasche (11) an einem ggf. nachträglich am Behälter (3) fixierbaren Grundkörper (9) schwenkbar angelenkt ist.

4. Behälterverschluss nach einem der vorangehenden Ansprüche,
**dadurch gekennzeichnet,**
**dass** die Schließlasche (11) und der behälterfeste Grundkörper eine Durchgangsbohrung (16,17) zum Durchtritt der Verschlussplombe aufweist.

5. Behälterverschluss nach einem der vorangehenden Ansprüche,
**dadurch gekennzeichnet,**
**dass** der Behälterrand querschnittlich als nach außen offenes U geformt ist, wobei der Grundkörper am U-Steg fixiert ist.

6. Behälterverschluss nach einem der vorangehenden Ansprüche,
**dadurch gekennzeichnet,**
**dass** der Grundkörper eine gestufte Lasche mit einer Durchtrittsbohrung für die Plombe aufweist, die außenrandseitig einen Fixiersteg für eine Klemmwulst an der schwenkbaren Schließlasche bildet.

7. Behälterverschluss nach Anspruch 6,
**dadurch gekennzeichnet,**
**dass** der die Klemmwulst tragende Fixiersteg als Abschlusswandung für an der Plombe vorgesehene Widerhaken ausgestaltet ist.

## Claims

1. A container closure, in particular for mounting a closure seal for safeguarding in the closed condition and which cannot be removed without being destroyed, in particular in relation to plastic containers, comprising a locking tongue (6), a through opening (8) associated with the locking tongue (6) in the upper edge region of the container, a pivotable closing tongue (11), wherein the closing tongue (11) has an engagement arm (12), and a seal through aperture (16),
**characterised in that**
the locking tongue (6) is arranged at the underside of the container top (2) in the edge region and the closing tongue (11) is provided in the edge region of the container and has an engagement arm (12) for engagement into a corresponding opening (5) of the locking tongue (6) and a clamping latch (13) for fixing the locking tongue (6) in the closed position, wherein the seal through aperture (16) is provided in the closing tongue (11).

2. A container closure according to claim 1 **characterised in that** the locking tongue (6) and/or the closing tongue (11) are in the form of independent components permanently fixable to the container elements (2, 3).

3. A container closure according to claim 2 **characterised in that** the closing tongue (11) is pivotably mounted to a main body (9) which is possibly subsequently fixable to the container (3).

4. A container closure according to one of the preceding claims **characterised in that** the closing tongue (11) and the main body which is fixed with respect to the container has a through bore (16, 17) for the closure seal to pass therethrough.

5. A container closure according to one of the preceding claims **characterised in that** the container edge is shaped in cross-section in the form of an outwardly open U, wherein the main body is fixed to the cross bar of the U.

6. A container closure according to one of the preceding claims **characterised in that** the main body has a stepped tongue with a through bore for the seal which at the outside edge side forms a fixing arm for a clamping ridge on the pivotable closing tongue.

7. A container closure according to claim 6 **characterised in that** the fixing arm carrying the clamping ridge is in the form of a cover wall for barb portions provided on the seal.

## Revendications

1. Obturateur de récipient, en particulier pour l'application d'un plombage de fermeture garantissant l'état de fermeture et ne pouvant pas être enlevé sans destruction, en particulier pour des récipients en matière plastique, comprenant une patte de blocage (6), un évidement traversant (8) associé à la patte de blocage (6) dans la zone de bordure supérieure du récipient, une patte de fermeture pivotante (11), ladite patte de fermeture (11) comprenant une barrette d'engagement (12), ainsi qu'une ouverture de traversée de plombage (16),
**caractérisé en ce que**
la patte de blocage (6) est agencée sur la face inférieure du couvercle (2) du récipient dans la zone de bordure, et **en ce que** la patte de fermeture (11) est prévue dans la zone de bordure du récipient et comprend une barrette d'engagement (12) pour l'engagement dans un évidement correspondant (5) de la patte de blocage (6), et un cran de coincement (13) pour fixer la patte de blocage (6) dans la position de fermeture, l'ouverture de traversée de plombage (6) étant prévue dans la patte de fermeture (11).

2. Obturateur de récipient selon la revendication 1,
**caractérisé en ce que** la patte de blocage (6) et/ou la patte de fermeture (11) sont réalisées sous forme de composants autonomes susceptibles d'être fixés durablement sur les éléments (2, 3) du récipient.

3. Obturateur de récipient selon la revendication 2,
**caractérisé en ce que** la patte de fermeture (11) est articulée de façon pivotante sur un corps de base (9) susceptible d'être fixé, éventuellement a posteriori, sur le récipient (3).

4. Obturateur de récipient selon l'une des revendications précédentes,
**caractérisé en ce que** la patte de fermeture (11) et le corps de base solidaire du récipient comportent un perçage traversant (16, 17) pour la traversée du plombage de fermeture.

5. Obturateur de récipient selon l'une des revendications précédentes,
**caractérisé en ce que** la bordure du récipient est formée, pour ce qui concerne sa section, sous la forme d'un U ouvert vers l'extérieur, le corps de base étant fixé sur la base du U.

6. Obturateur de récipient selon l'une des revendications précédentes,
**caractérisé en ce que** le corps de base comprend une patte en gradins avec un perçage traversant pour le plombage, qui forme du côté de sa bordure extérieure une barrette de fixation pour un bourrelet de coincement sur la patte de fermeture pivotante.

7. Obturateur de récipient selon la revendication 6,
**caractérisé en ce que** la barrette de fixation portant le bourrelet de coincement est conçue à titre de paroi de fermeture pour des ardillons prévus sur le plombage.
